# EUROPEAN PATENT SPECIFICATION

(11) **EP 1 188 836 B1**
(45) Date of publication and mention of the grant of the patent: **04.06.2008**
(21) Application number: 01120988.9
(22) Date of filing: 31.08.2001
(51) Int. Cl.: C12P 7/62, C12N 1/28

(54) **Production method of polyester containing epoxy group in side chain and production method of crosslinked polymer**
Herstellungsmethode von Polyestern mit epoxidgruppenhaltigen-Seitenketten und Herstellungsmethode von vernetzten Polymeren
Résine de polyestère contenant un groupe époxy dans les chaînes latérales et polymère réticulé et procédés de préparation

(30) Priority: 31.08.2000 JP 2000263508; 27.09.2000 JP 2000294635
(43) Date of publication of application: 20.03.2002
(73) Proprietor: CANON KABUSHIKI KAISHA, Ohta-ku, Tokyo (JP)
(72) Inventor: Imamura, Takeshi, Tech.-Ing., Ohta-ku, Tokyo (JP); Sugawa, Etsuko, Tech.-Ing., Ohta-ku, Tokyo (JP); Yano, Tetsuya, Tech.-Ing., Ohta-ku, Tokyo (JP); Kobayashi, Shin, Tech.-Ing., Ohta-ku, Tokyo (JP); Honma, Tsutomu, Tech.-Ing., Ohta-ku, Tokyo (JP); Kenmoku, Takashi, Tech.-Ing., Ohta-ku, Tokyo (JP)
(74) Representative: Weser, Wolfgang

(56) References cited:
- EP-A- 0 274 151
- EP-A- 1 113 076
- LAGEVEEN R G ET AL: "FORMATION OF POLYESTERS BY PSEUDOMONAS OLEOVORANS: EFFECT OF SUBSTRATES ON FORMATION AND COMPOSITION OF POLY-(R)-3- HYDROXYALKANOATES AND POLY-(R)-3-HYDROXYALKENOATES" APPLIED AND ENVIRONMENTAL MICROBIOLOGY, WASHINGTON,DC, US, vol. 54, no. 12, 1 December 1988 (1988-12-01), pages 2924-2932, XP000106590 ISSN: 0099-2240
- LEE M Y ET AL: "Epoxidation of bacterial polyesters with unsaturated side chains V. Effect of crosslinking on thermal degradation of epoxidized polymers" POLYMER DEGRADATION AND STABILITY, BARKING, GB, vol. 65, no. 1, July 1999 (1999-07), pages 137-142, XP004294687 ISSN: 0141-3910
- PARK W H ET AL: "Epoxidation of bacterial polyesters with unsaturated side chains: III. Crosslinking of epoxidized polymers" POLYMER DEGRADATION AND STABILITY, BARKING, GB, vol. 36, 1998, pages 2389-2396, XP002188518 ISSN: 0141-3910
- PARK W H ET AL: "Epoxidation of bacterial polyesters with unsaturated side chains: II. Rate of epoxidation and polymer properties" POLYMER DEGRADATION AND STABILITY, BARKING, GB, vol. 36, 1998, pages 2381-2387, XP002188519 ISSN: 0141-3910
- PARK W H ET AL: "Epoxidation of bacterial polyesters with unsaturated side chains: IV. Thermal degradation of initial and epoxidized polymers" POLYMER DEGRADATION AND STABILITY, BARKING, GB, vol. 63, no. 2, February 1999 (1999-02), pages 287-291, XP004294600 ISSN: 0141-3910
- PARK W H ET AL: "EPOXIDATION OF BACTERIAL POLYESTERS WITH UNSATURATED SIDE CHAINS. I. PRODUCTION AND EPOXIDATION OF POLYESTERS FROM 10-UNDECENOIC ACID" MACROMOLECULES, AMERICAN CHEMICAL SOCIETY. EASTON, US, vol. 31, no. 5, 10 March 1998 (1998-03-10), XP002188520 ISSN: 0024-9297

## Description

### BACKGROUND OF THE INVENTION

### Field of the Invention

The present invention relates to a method of producing a polyester using a microorganism.

### Related Background Art

So far, it has been reported that a variety of microorganisms produce poly(3-hydroxybutyrate) (hereinafter, abbreviated as PHB) or other polyhydroxyalkanoates (PHA) and store it in their bodies ("Biodegradable plastic handbook", Biodegradable Plastic Study Associate edition, N.T.S Co., Ltd., pp. 178-197, 1995). These polymers can be utilized for production of various types of products by melt processing or the like, as is the case with conventional plastics. Further, they are biodegradable and therefore have an advantage that they can completely be decomposed by microorganisms in nature, and unlike conventional many synthetic polymeric compounds, they do not remain in natural environments to cause environmental pollution and may not generate harmful substances such as dioxins, endocrine disrupting chemical substances, etc. since they are not required to be incinerated. Furthermore, they are excellent in biocompatibility and highly expected to be applied to the use as soft members for medical care (Japanese Patent Application Laid-Open No. 5-000159).

Recently, in the industrial application of such PHA, it has been attempted to extend the diversity in the physicochemical characteristics of PHA by producing PHA composed of units different from common monomer units.

As one of such methods, an attempt has been made to improve the physicochemical properties of PHA by introducing epoxy groups in side chains of PHA and carrying out a crosslinking reaction or chemical modification using the introduction sites as active points.

There is reported in Macromolecules, 31, pp. 1480-1486 (1998) and Journal of Polymer Science: Part A: Polymer Chemistry, 36, pp. 2381-2387 (1998), synthesis of PHA containing epoxy groups in the side chain terminals by culturing Pseudomonas oleovorans in culture media containing sodium octanoate and 10-undecenoic acid as an unsaturated fatty acid in various ratios to produce PHA containing a variety of percentages of units with unsaturated bonds in the terminals of the side chains and then chemically epoxidizing the unsaturated sites with 3-chlorobenzoic acid. Further, there is reported in Journal of Polymer Science: Part A: Polymer Chemistry, 36, pp. 2389-2396 (1998) that a crosslinking reaction of the above described epoxy PHA was carried out with succinic anhydride using 2-ethyl-4-methylimidazole as an initiator.

As described above, in the improvement of the physicochemical properties of PHA, epoxy groups of the side chain terminals are very useful, however, there is no synthesis method other than the chemical epoxidation of the unsaturated sites in the side chain terminals, and such chemical epoxidation requires very complicated operations and has therefore a practical disadvantage in terms of cost.

### SUMMARY OF THE INVENTION

It is, therefore, an object of the present invention to provide a method for solving the above described problems.

According to a first aspect of the present invention, there is provided a method of producing a polyester that contains an epoxy group in a side chain thereof using 1-alkene having 7 to 12 carbon atoms as a raw material, comprising the steps of bringing 1-alkene into contact with a microorganism having an ability to uptake 1-alkene and convert it to a polyester and allowing the microorganism to convert the 1-alkene into a polyester containing an epoxy group in a side chain thereof.

In the present invention, it is preferred that the method comprises the step of culturing the microorganism in a culture medium containing the 1-alkene.

In the present invention, it is also preferred that the method further comprises the step of isolating the polyester produced by the microorganism.

In the present invention, it is further preferred that the isolation step comprises recovering the polyester from the cell of the microorganism.

According to a second aspect of the present invention, there is provided a method of producing a crosslinked polymer comprising reacting the polyester obtained by the above mentioned method with a diamine compound.

### BRIEF DESCRIPTION OF THE DRAWINGS

FIG. 1 is a graphical representation showing 1H-NMR of the polymer obtained in Example 1;
FIG. 2 is a graphical representation showing 1H-NMR of the polymer obtained in Example 2;
FIG. 3 is a graphical representation showing 1H-NMR of the polymer obtained in Example 3;
FIG. 4 is a graphical representation showing 1H-NMR of the polymer obtained in Example 4;
FIG. 5 is a graphical representation showing 1H-NMR of the polymer obtained in Example 5;
FIG. 6 is a graphical representation showing 1H-NMR of the polymer obtained in Example 6;
FIG. 7 is a scheme showing the routes of polymer production from 1-alkene using YN2 strain;
FIGS. 8A, 8B and 8C are views each showing a GC chart of the result described in Example 7;
FIGS. 9A, 9B and 9C are views each showing a GC chart of the result described in Example 8;
FIG. 10 is a graphical representation showing a DSC chart of the polymer described in Example 9; and
FIGS. 11A and 11B are graphical representations each showing a FT-IR chart of the polymer described in Example 9.

### DETAILED DESCRIPTION OF THE PREFERRED EMBODIMENTS

The polyester obtained according to the method of the present invention contains at least 1 mol % of a unit represented by the chemical formula (1): 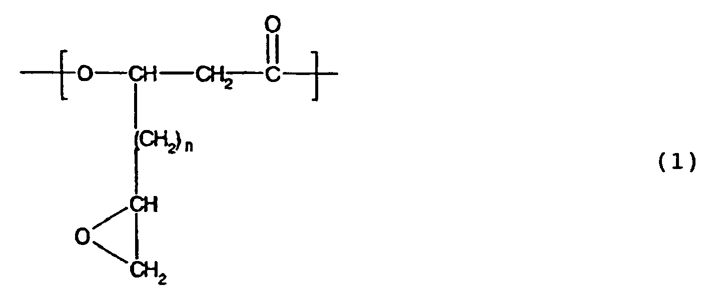 (wherein n is an integer of 1 to 7) in monomer units thereof.

The polyester obtained according to the method of the present invention may further contain at least 1 mol % of a unit represented by the chemical formula (2): 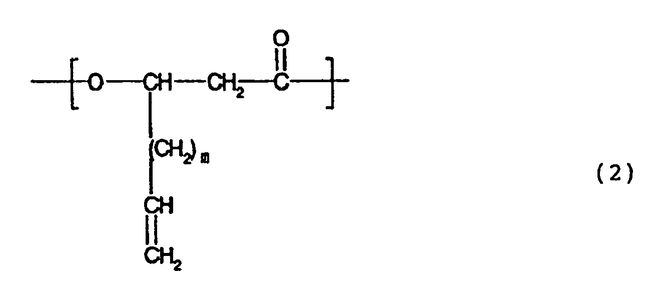 (wherein m is an integer of 1 to 7) in monomer units thereof.

The polyester obtained according to the method of the present invention may further contain at least 1 mol % of a unit represented by the chemical formula (3): 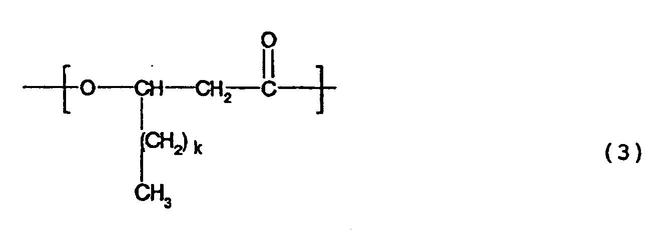 (wherein k is an integer of 0 to 8) in monomer units thereof.

The 1-alkene to be used as a raw material in the method of the present invention is preferably an 1-alkene with 7 to 12 carbons, namely 1-heptene, 1-octene, 1-nonene, 1-decene, 1-undecene, and 1-dodecene.

Further, the number-average molecular weight of the polyester obtained by the present invention is 10,000 to 1,000,000 and more particularly 10,000 to 500,000.

### <Microorganism>

The microorganism to be used for the method of the present invention is a microorganism having an ability to epoxidize the 1-alkene and convert it to an corresponding epoxyalkane compound; an ability to convert a terminal of the epoxyalkane compound to form an epoxidized carboxylic acid; and an ability to convert the epoxidized carboxylic acid to a polyester and includes microorganisms belonging to Pseudomonas species and more particularly includes Pseudomonas cichorii YN2 strain; FERM BP-7375 used in the examples of the present invention as described below.

Pseudomonas cichorii YN2; FERM BP-7375 as a microorganism used for the present invention is a microorganism having the following properties and deposited to International Patent Organism Depositary in National Institute of Advanced Industrial Science and Technology, AIST (deposition number: FERM BP-7375).

The mycological properties of the YN2 strain are as follows.

### (1) Morphological properties

culture temperature: 30°C
cell shape: rod, 0.8 µm × 1.5 to 2.0 µm
Gram staining: negative
sporulation: negative
motility: positive
colony shape: circular; entire, smooth margin; low convex; smooth surface; glossy; translucent

### (2) Physiological properties

catalase: positive
oxidase: positive
O/F test: non-fermentative
nitrate reduction: negative
indole production: positive
glucose oxidation: negative
arginine dihydrolase: negative
urease: negative
esculin hydrolysis: negative
gelatin hydrolysis: negative
β-galactosidase: negative
fluorescent pigment production on King's B agar: positive
growth under 4% NaCl: positive (weak growth)
poly-p-hydroxybutyrate accumulation: negative(*)
Tween 80 hydrolysis: positive
* determined by staining colonies cultured on nutrient agar with Sudan Black '

### (3) Substrate Assimilation

glucose: positive
L-arabinose: positive
D-mannose: negative
D-mannitol: negative
N-acetyl-D-glucosamine: negative
maltose: negative
potassium gluconate: positive
n-caprate: positive
adipate: negative
dl-malate: positive
sodium citrate: positive
phenyl acetate: positive

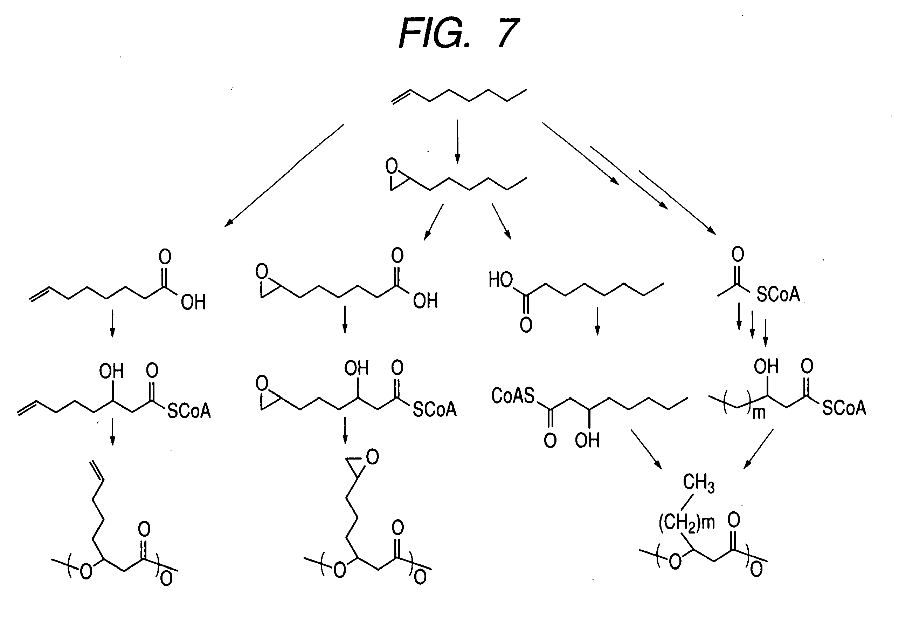

This bacterial strain is also a microorganism disclosed in Japanese Patent Application No. 11-371863. This bacterial strain has a capability of epoxidizing 1-alkene to an corresponding epoxyalkane as will be described in the examples below. Generally, the enzyme for exhibiting such a capability is an alkene-monooxygenase. It is highly probable that this bacterial stain also has the alkene-monooxygenase. Further, this bacterial strain has not been found to produce an epoxyalkanoic acid from a corresponding alkenoic acid. Based on the results deduced from the above described matter, it is implied that the route of the polyester production of the present invention by this bacterial stain is those shown in FIG. 7.

### <Culture process>

Any culture may be usable as a culture to be employed for the present invention as long as it is an inorganic salt culture containing phosphorate and a nitrogen source such as an ammonium salt or a nitrate and it is possible to improve the productivity of PHA by adjusting the concentration of the nitrogen source. Since a 1-alkene to be added has a low solubility in water and is highly volatile, it is required to supply the 1-alkene in a gas state during the culture and to put it in sealed state while ensuring oxygen which the microorganism requires.

The composition of a culture employed for one embodiment of the method of the present invention as an example of an inorganic salt culture is shown below.

### (M9 culture)

Na₂HPO₄: 6.3
KH₂PO₄: 3.0
NH₄Cl: 1.0
NaCl: 0.5 g/L, pH = 7.0

### (1/10N-M9 culture)

Na₂HPO₄: 6.3
KH₂PO₄: 3.0
NH₄Cl: 0.1
NaCl: 0.5 g/L, pH = 7.0

Further, in order to maintain good prolification and PHA productivity, it is required to add the following solution of the trace amount components in about 0.3% (v/v) to the above described inorganic salt culture:

### (Trace amount component solution)

nitrilo triacetate: 1.5; MgSO₄: 3.0;
MnSO₄: 0.5; NaCl: 1.0;
FeSO₄: 0.1; CaCl₂: 0.1;
COCl₂: 0.1; ZnSO₄: 0.1;
CuSO₄: 0.1; AlK(SO₄)₂: 0.1;
H₃BO₃: 0.1; Na₂MoO₄: 0.1; and
NiCl₂: 0.1 (unit: g/L)

The culture temperature may be any temperature at which good prolification of the above described bacterial strain can be assured and it is preferably about 20° C to 30° C.

Any culture method including a liquid culture method, a solid culture method, etc. can be employed as long as it is suitable for prolification of the microorganism and production of PHA. Further, the type of the culture includes, but are not limited to, a batch culture, a fed-batch culture, a semi-continuous culture, and a continuous culture.

A commonly employed method can be employed for obtaining PHA from the culture substances containing cultured cells of the present invention and the culture liquid. In the case where PHA is secreted into the culture liquid, a method for extraction and purification from the culture liquid is employed and in the case where PHA is accumulated in the cells, a method for extraction and purification from the cells is employed. For example, for recovering PHA from the cultured cells of the microorganism, chloroform extraction, which is commonly employed, is most convenient, however in the environments where an organic solvent is troublesome to be used, there can be employed a method of recovering only PHA by removing other components in cells other than PHA by treatment with a surfactant such as SDS, etc., treatment with an enzyme such as lysozyme, etc., treatment by chemicals such as EDTA, sodium hypochlorite, ammonia, etc.

Incidentally, there is reported in Appl. Environ. Microbiol., 54, pp. 2924-2932 (1998) production of a polyester using Pseudomonas oleovorans similar to the method of the present invention, however the polyester produced therein has no epoxy groups in the side chains but contains both units having double bonds in terminals of side chains and units having saturated alkylene chains as side chains.

The polymer obtained according to the method of the present invention can be subjected to chemical conversion, as with common polymers having epoxy groups. More particularly, the chemical conversion includes a crosslinking reaction with hexamethylenediamine, succinic anhydride, or 2-ethyl-4-methylimidazole, or electron beam irradiation. Further, it is also possible to convert it into hydroxyl groups or to introduce sulfone groups thereinto. Furthermore, it is also possible to add a compound having thiol or amine thereto.

The present invention further provides a method of producing a crosslinked polymer by reacting the above mentioned polyester with a diamine compound. More particularly, the present invention provides a method of producing a crosslinked polymer by reacting the above mentioned polyester with hexamethylenediamine. Such a reaction proceeds along a reaction route as shown in the following scheme to produce a crosslinked polymer.

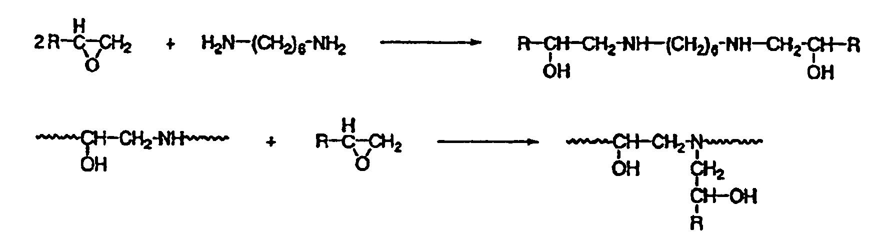

The reaction temperature is preferably 50°C to 120°C and the reaction time is preferably within the range of 10 minutes to 120 minutes.

### <Examples>

Now, examples will be described, but the present invention is not limited to the examples.

### (Example 1)

Colonies of YN2 strain on the M9 agar culture containing 0.1% of yeast extract were suspended in a physiological saline solution so sterilized as to have OD (600 nm) = 1.0. The resulting suspension was applied to 20 plates of 1/10N-M9 agar cultures free from C sources and static cultivation was carried out at 30°C in a 1-heptene atmosphere.

After 4 days, cells were combined together, cleaned with methanol, collected by centrifugal separation, and dried in vacuum.

To the dried cells, 50 mL of chloroform was added and stirred at 30°C for 48 hours to extract PHA. The chloroform layer was then filtered and concentrated by an evaporator, which was then added to cold methanol and the precipitate was recovered and dried in vacuum.

### (Example 2)

A production experiment was carried out in the same manner as in Example 1 except that 1-heptene was changed to 1-octene.

### (Example 3)

A production experiment was carried out in the same manner as in Example 1 except that 1-heptene was changed to 1-nonene.

### (Example 4)

A production experiment was carried out in the same manner as in Example 1 except that 1-heptene was changed to 1-decene.

### (Example 5)

A production experiment was carried out in the same manner as in Example 1 except that 1-heptene was changed to 1-undecene.

### (Example 6)

A production experiment was carried out in the same manner as in Example 1 except that 1-heptene was changed to 1-dodecene.

The weights of the cells and dried polymers obtained in Example 1 to 6 were shown in Table 1 below.

**Table 1**

| Example No. | Dry weight of cells (mg) | Dry weight of polymer (mg) |
|---|---|---|
| 1 | 160 | 48 |
| 2 | 170 | 52 |
| 3 | 160 | 55 |
| 4 | 180 | 58 |
| 5 | 170 | 55 |
| 6 | 160 | 48 |

### (Analysis and Evaluation)

Analysis of the units of the polymer obtained in Examples 1 to 6 was carried out as follows. That is, about 10 mg of PHA was put in an eggplant type flask of 25 mL capacity and dissolved in 2 mL of chloroform, and 2 mL of a methanol solution containing 3% of sulfuric acid was added thereto and a reaction was effected at 100°C for 3.5 hours under reflux. After completion of the reaction, 10 mL of deionized water was added and the resulting mixture was shaken vigorously for 10 minutes, and an underlying chloroform layer of two separated layers was taken out, dehydrated with magnesium sulfate and subjected to a gas chromatographic mass spectrograph (GC-MS, Shimadzu QP-5050 model, EI method) to identify the methyl ester of PHA monomer units. The results of area % of total ion chromatogram (TIC) were shown in Table 2. In this case, since the monomer units were converted by methanolysis, no epoxy unit was detected.

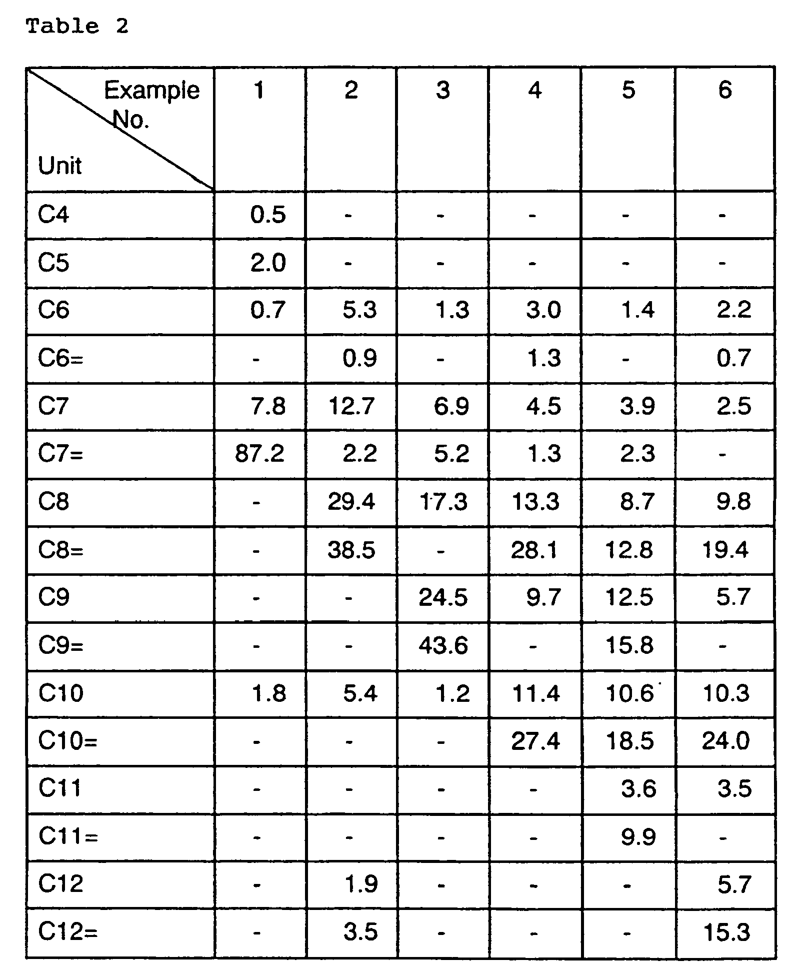

In table 2, the symbols used for representing the units have the following meaning.
C4: 3-hydroxybutyric acid; C5: 3-hydroxyvaleric acid; C6: 3-hydroxyhexanoic acid; C6=: 3-hydroxy-5-hexenoic acid; C7: 3-hydroxyheptanoic acid; C7=: 3-hydroxy-6-heptenoic acid; C8: 3-hydroxyoctanoic acid; C8=: 3-hydroxy-7-octenoic acid; C9: 3-hydroxynonanoic acid; C9=: 3-hydroxy-8-nonenoic acid; C10: 3-hydroxydecanoic acid; C10=: 3-hydroxy-9-decenoic acid; C11: 3-hydroxyundecanoic acid; C11=: 3-hydroxy-10-undecenoic acid; C12: 3-hydroxydodecanoic acid; and C12=: 3-hydroxy-11-dodecenoic acid.

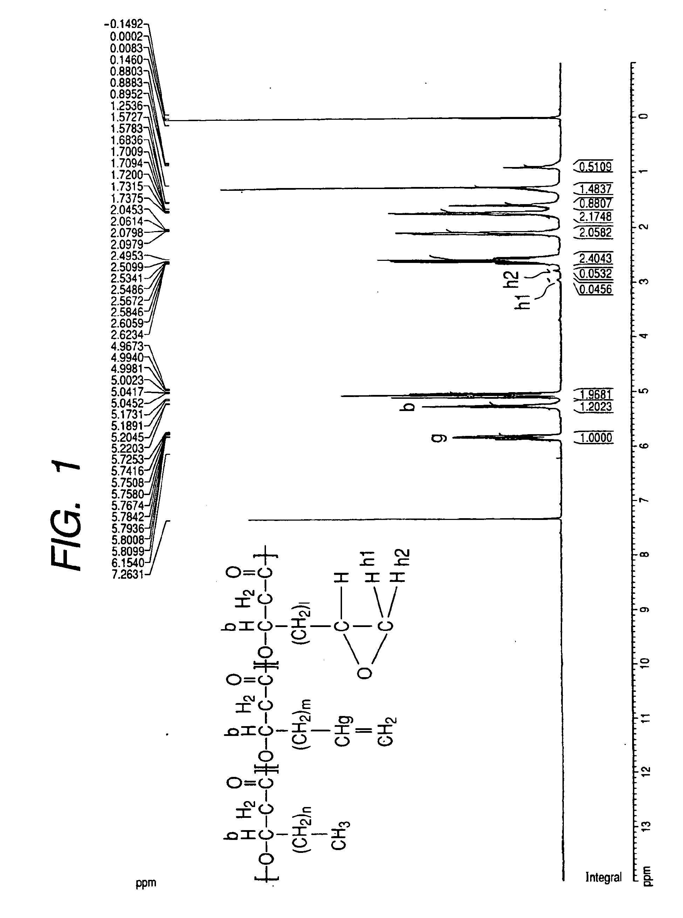

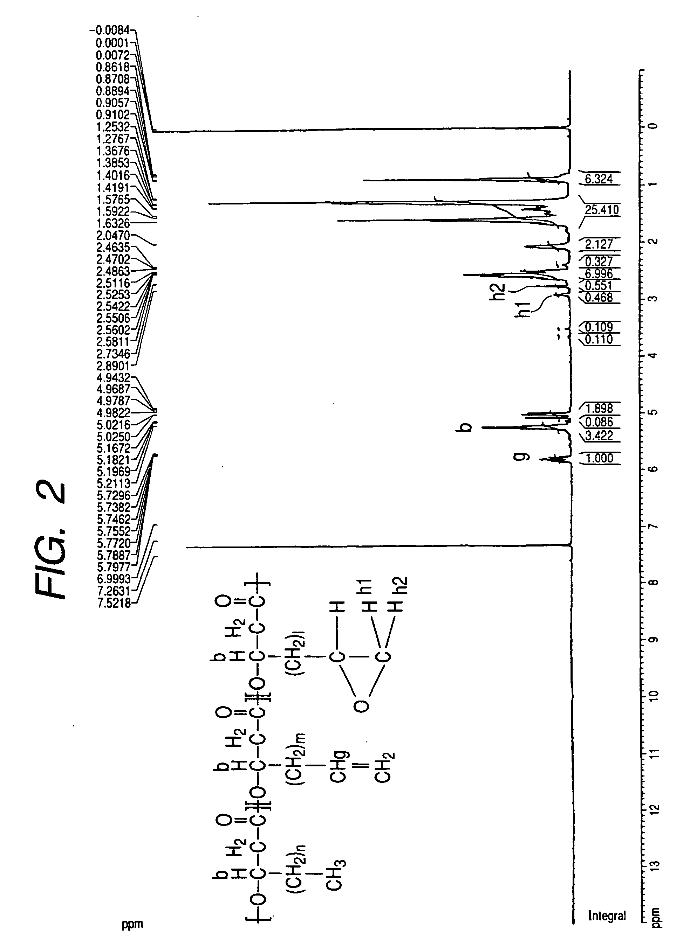

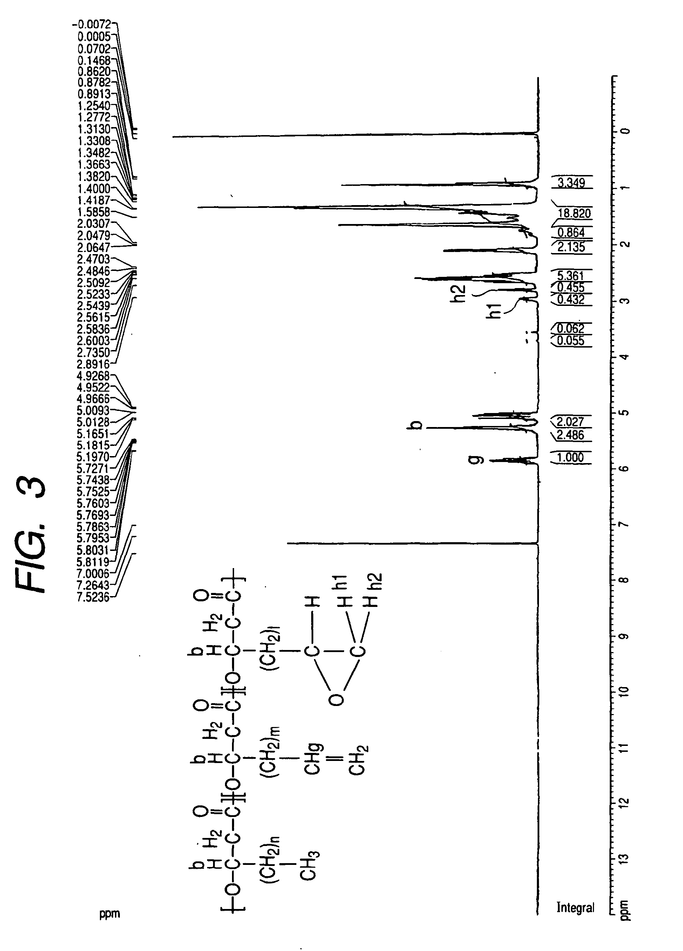

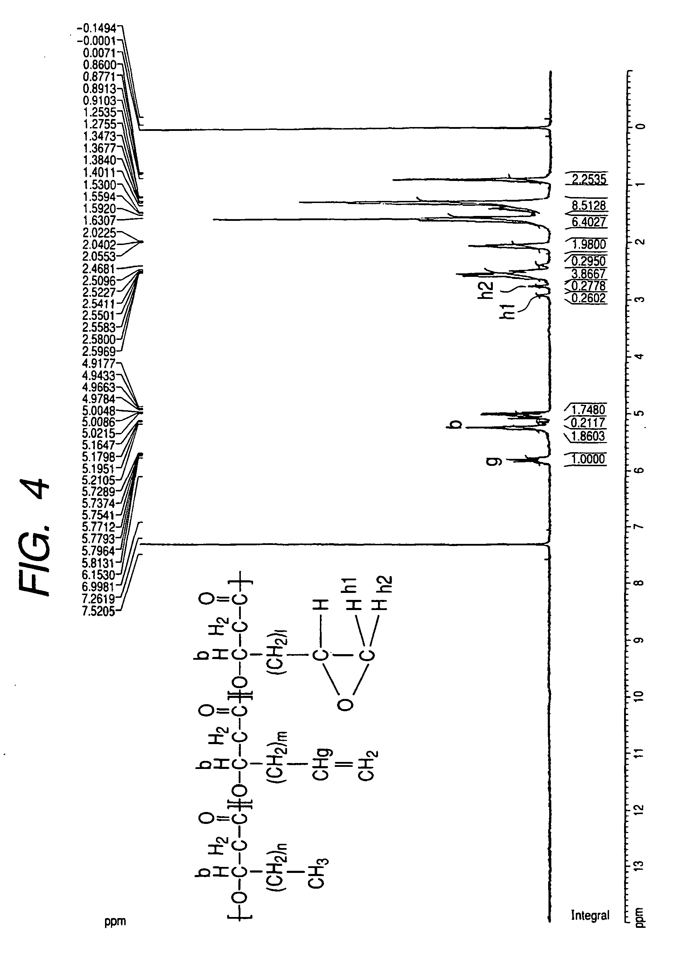

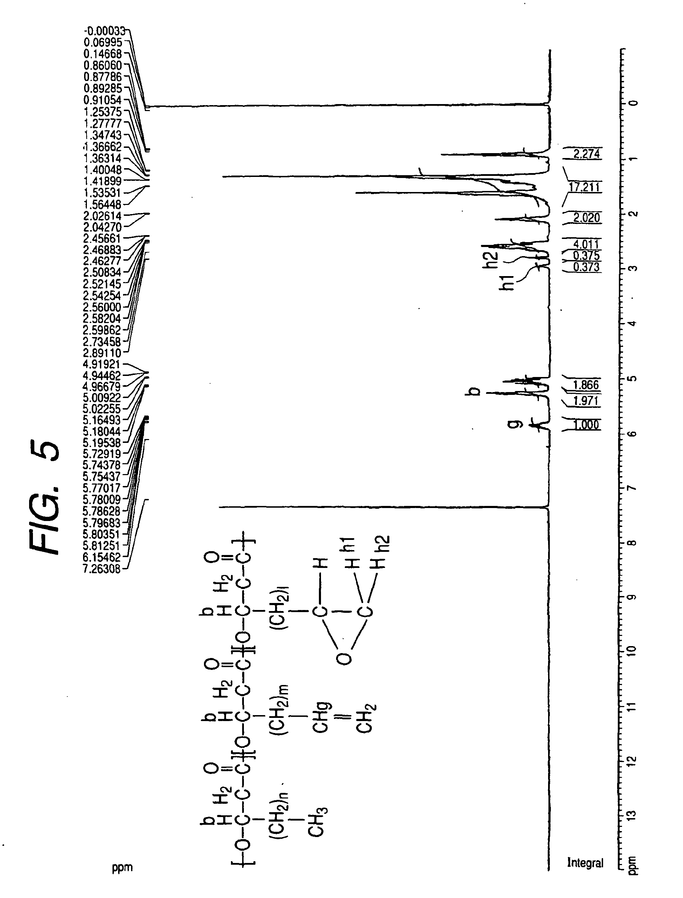

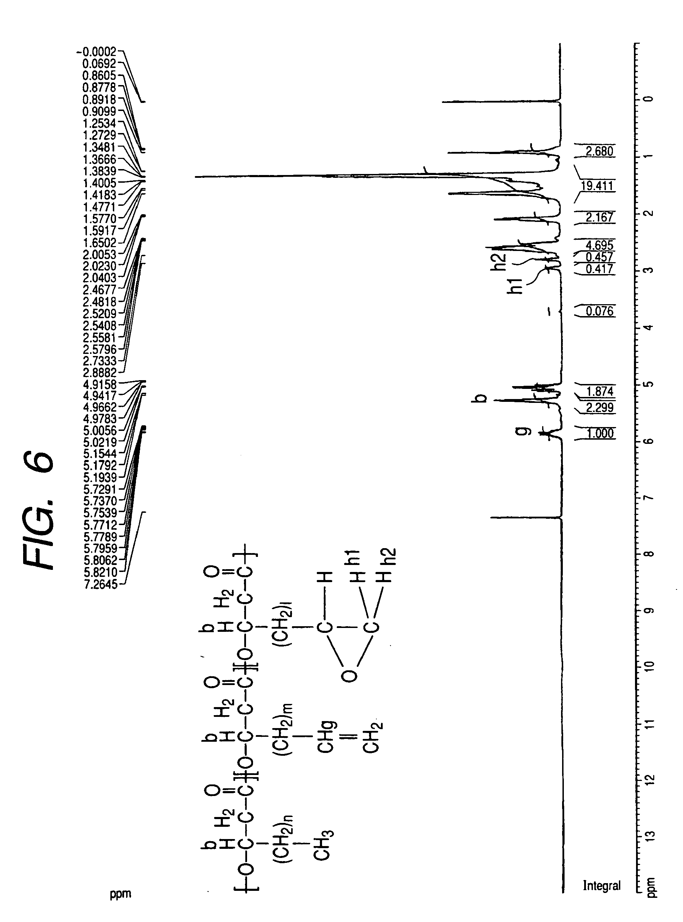

The polymers obtained in Examples 1 to 6 were subjected to 1H-NMR analysis (Analyzer: FT-NMR: Bruker DPX400; Determined nuclide: 1H; Solvent used: dichloroform with TMS). The attribution of protons of methine in side chain terminals, double bonds in side chain terminals, and epoxy groups was determined according to the method described in Macromolecules, 31, pp. 1480-1486 (1998). The spectra thus obtained were shown in FIGS. 1 to 6.

The mol % of respective side chain units (saturated terminal, unsaturated (double-bonded) terminal, and epoxidized terminal) calculated based on the above described results were shown in Table 3.

**Table 3**

| Carbon source | Monomer units (mol %)* | | | |
|---|---|---|---|---|
| | Saturated groups | Terminal unsaturated groups | Other epoxidized groups | Unsaturated groups |
| Hexene | 70.0 | 20.0 | ND** | 10.0 |
| Heptene | 12.5 | 83.3 | 4.2 | ND |
| Octene | 55.9 | 29.4 | 14.7 | ND |
| Nonene | 44.0 | 40.0 | 16.0 | ND |
| Decene | 31.6 | 52.6 | 15.8 | ND |
| Undecene | 30.0 | 50.0 | 20.0 | ND |
| Dodecene | 39.1 | 43.5 | 17.4 | ND |

| | | | | |
|---|---|---|---|---|
| Note: The mol % of the monomer units was identified by integration with ¹H-NMR. ** ND means "not detected". | | | | |

Further, the molecular weights of the polymers obtained in Examples 1 to 6 were evaluated by GPC (Tosoh Corporation HLC-8020; Column: Polymer Laboratory, PL gel MIXED-C (5 µm); Solvent: chloroform; Converred on basis of polystyrene). The results were shown in Table 4.

**Table 4**

| Example | Number-average molecular weight (Mn) × 10⁵ | Weight-average molecular weight (Mw) × 10⁵ |
|---|---|---|
| 1 | 1.9 | 5.2 |
| 2 | 2.5 | 5.5 |
| 3 | 2.6 | 5.3 |
| 4 | 1.9 | 5.4 |
| 5 | 1.9 | 5.4 |
| 6 | 2.0 | 4.9 |

### (Example 7)

YN2 strain was cultured at 30°C for 24 hours in a culture medium containing 0.5% polypeptone, and the cells were collected by centrifugal separation and again suspended in an inorganic salt culture medium. 10 mL of the resulting cell suspension was put in a vial of 27 mL capacity and sealed with a butyl rubber plug and an aluminum seal, and air containing 1-hexene gas was added thereto with a syringe. As a control, a sample only of an inorganic salt culture medium containing no YN2 strain was prepared in the same manner and the respective vials were shaken at 30°C for 1 hour. After the shaking, 0.1 mL of a vapor phase in each vial was withdrawn by a syringe and subjected to a gas chromatographic (GC) analysis. The conditions of the GC were as follows.

Analyzer: Shimadzu GC-14B; Column: DB-624 (mfd. by J & W Co.); Column temperature: constantly 100°C; Injector /detector temperature: 230°C; Detector: FID

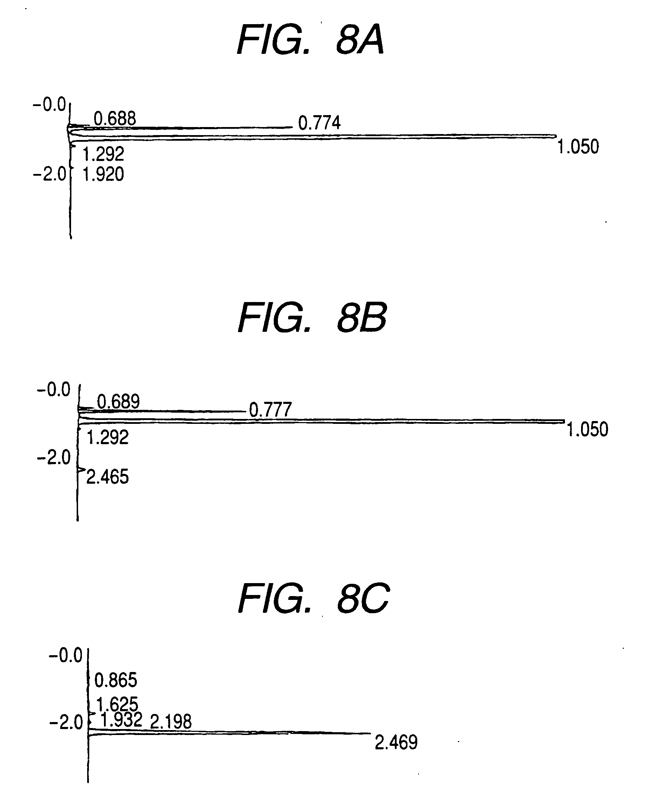

The results are shown in FIGS. 8A to 8C. FIG. 8A shows the results of the sample only of the inorganic salt culture medium containing no YN2 strain. A peak of 1-hexene is observed near 1.05. FIG. 8B shows the results of the sample of the cell suspension of YN2 strain. A peak, which is not observed in FIG. 8A, is observed near 2.47. FIG. 8C shows the results of a sample of a standard sample of 1,2-epoxyhexane. A peak corresponding to the above mentioned peak is observed near 2.47. According to the results, it was made clear that the YN2 strain converted 1-hexene to 1,2-epoxyhexane.

### (Example 8)

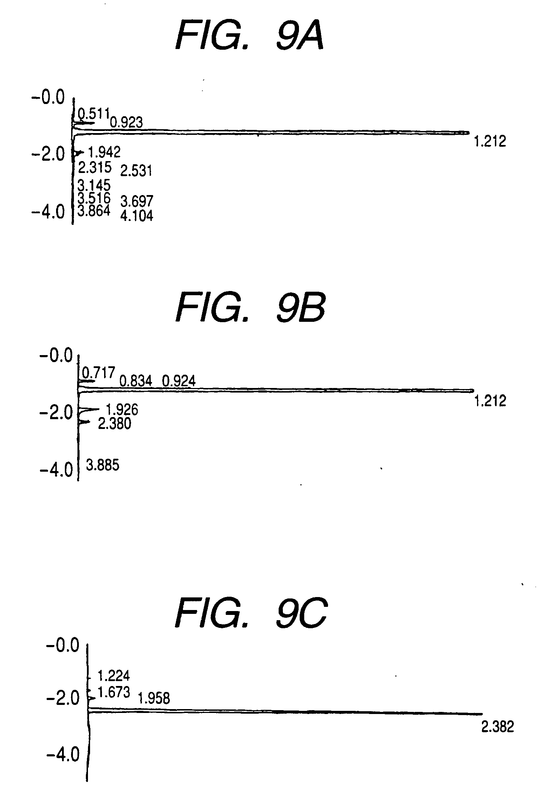

The conversion activity of YN2 strain to 1-octene was evaluated in the same manner as in Example 7 (GC column temperature: 150°C). The results are shown in FIGS. 9A to 9C. FIG. 9A shows the results of the sample only of the inorganic salt culture medium containing no YN2 strain. A peak of 1-octene is observed near 1.21. FIG. 9B shows the results of the sample of the cell suspension of YN2 strain. A peak, which is not observed in FIG. 9A, is observed near 2.38. FIG. 9C shows the results of a sample of a standard sample of 1,2-epoxyoctane. A peak corresponding to the above mentioned peak is observed near 2.38. According to the results, it was made clear that the YN2 strain converted 1-octene to 1,2-epoxyoctane.

In other words, according to the results of Examples 7 and 8, it was made clear that YN2 strain has an ability to epoxidize 1-alkene to corresponding 1,2-epoxyalkane.

### (Example 9)

20 mg of the polymer obtained in Example 4 was dissolved in 0.2 mL of chloroform, and 10 mg of hexamethylenediamine was added thereto with cooling by ice to dissolve it. After completion of the dissolution was confirmed, chloroform was removed and then the resulting solution was subjected to a measurement with a differential scanning calorimeter (DSC; Pyris 1 mfd. by Perkin Elmer Co.; Temperature rise rate: 10°C/min). Further, another sample subjected to a reaction at 90°C for 1 hour was similarly subjected to the DSC measurement.

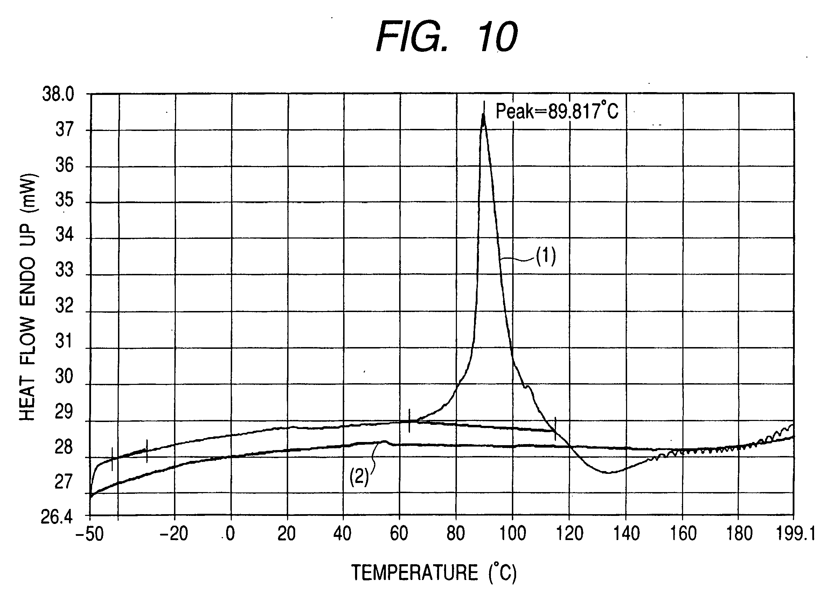

The results were shown in FIG. 10. In the figure, the chart shown by (1) is of the former sample (obtained only by mixing) and the chart shown by (2) is of the latter sample (further subjected to the reaction at 90°C for 1 hour). A clear heat generation peak was observed at near 90°C in the chart (1), which indicates that a reaction of the epoxy groups of the polymer obtained in Example 4 with hexamethylenediamine occurs and crosslinking between polymers proceeds. On the other hand, no clear heat flow is observed in the chart (2), indicating completion of the crosslinking reaction.

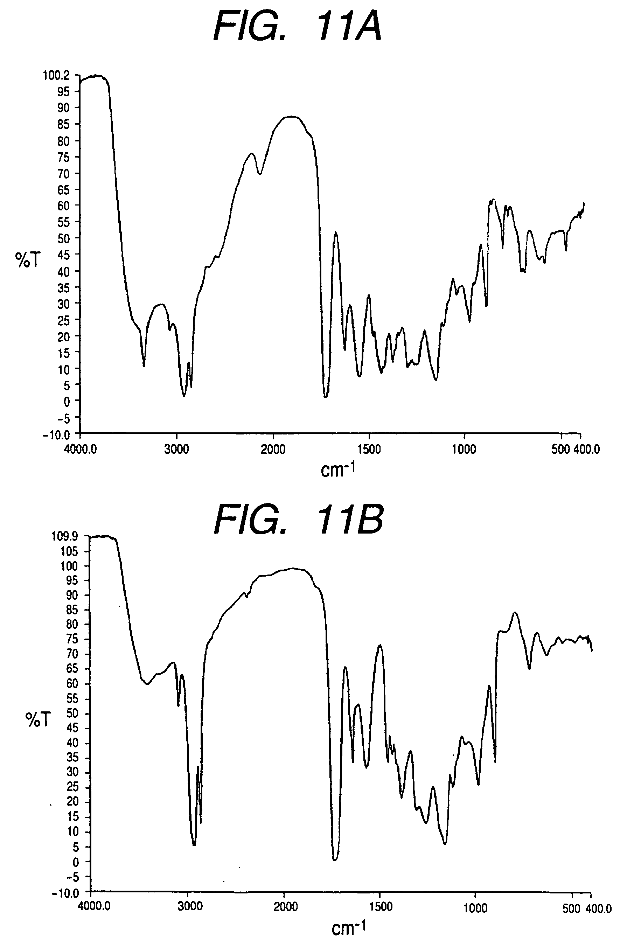

Further, using the same samples, IR absorption was measured (FT-IR; mfd. by Perkin Elmer Co., 1720X model). The results are shown in FIGS. 11A and 11B. The peak (near 3340 cm⁻¹) corresponding to amine and the peak (near 822 cm⁻¹) corresponding to epoxy group as observed in the chart of FIG. 11A disappear in the chart of FIG. 11B.

According to the above described results, it was made clear that a crosslinked polymer could be obtained by reacting, with hexamethylenediamine, a polyester having epoxy units in the side chains which was obtained by the method comprising the steps of bringing 1-alkene into contact with a microorganism having an ability to uptake and convert 1-alkene to a polyester and allowing the microorganism to convert the 1-alkene into a polyester.

## Claims

1. A method of producing a polyester containing an epoxy group in a side chain thereof using 1-alkene as a raw material, comprising the steps of
bringing 1-alkene into contact with a microorganism having an ability to uptake 1-alkene and convert it to a polyester and
converting the 1-alkene into a polyester containing an epoxy group in a side chain thereof by the microorganism,
wherein the 1-alkene has 7 to 12 carbon atoms.

2. The method according to claim 1, wherein the microorganism has
(a) an ability to epoxidize and convert the 1-alkene to an epoxyalkane compound;
(b) an ability to convert the epoxyalkane compound to an epoxidized carboxylic acid; and
(c) an ability to convert the epoxidized carboxylic acid to the polyester.

3. The method according to claim 1, further comprising the step of culturing the microorganism in a culture medium containing the 1-alkene.

4. The method according to claim 3, further comprising the step of isolating the polyester produced by the microorganism.

5. The method according to claim 4, wherein the isolation step comprises recovering the polyester from the cell of the microorganism.

6. The method according to claim 1, wherein the polyester contains at least 1 mol % of a unit represented by the chemical formula (1): 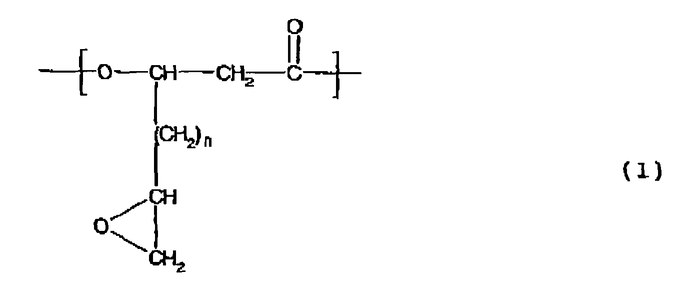 (wherein n is an integer of 1 to 7) in monomer units thereof.

7. The method according to claim 6, wherein the polyester contains at least 1 mol % of a unit represented by the chemical formula (2): 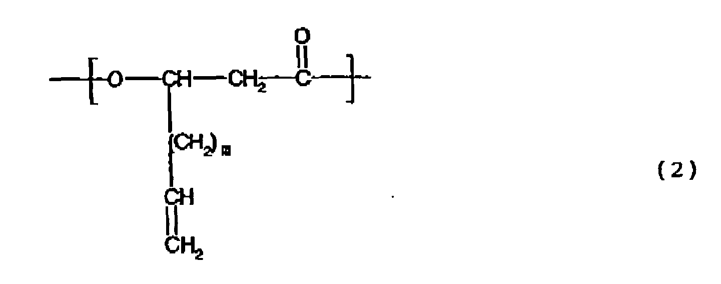 (wherein m is an integer of 1 to 7) in monomer units thereof.

8. The method according to claim 6, wherein the polyester contains at least 1 mol % of a unit represented by the chemical formula (3): 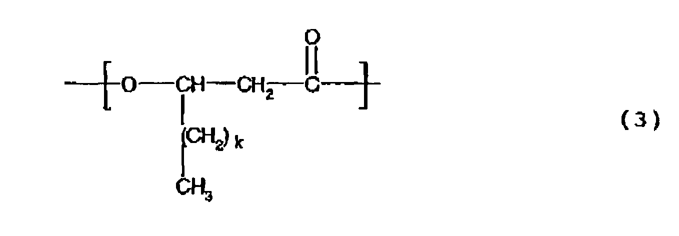 (wherein k is an integer of 0 to 8) in monomer units thereof.

9. The method according to claim 1, wherein the polyester has a number-average molecular weight of 10,000 to 1,000,000.

10. The method according to claim 1, wherein the microorganism belongs to Pseudomonas species.

11. The method according to claim 10, wherein the microorganism is Pseudomonas cichorii YN2; FERM BP-7375.

12. A method of producing a crosslinked polymer comprising a step of producing a polyester according to the method of claim 1 and a step of crosslinking the polyester obtained thereby by reacting the polyester with a diamine compound.

13. The method according to claim 12, wherein the diamine compound is hexamethylenediamine.

14. The method according to claim 12, wherein the reaction is carried out at a temperature within the range of 50°C to 120°C.

15. The method according to claim 12, wherein the reaction is carried out for 10 minutes to 120 minutes.

## Patentansprüche

1. Verfahren zur Herstellung eines Polyesters, der in einer Seitenkette eine Epoxygruppe enthält, unter Verwendung von 1-Alken als Ausgangsmaterial, umfassend die Schritte:
Inkontaktbringen von 1-Alken mit einem Mikroorganismus mit der Fähigkeit, 1-Alken aufzunehmen und dieses in einen Polyester zu überführen, und
Überführen des 1-Alkens durch den Mikroorganismus in einen Polyester, der in einer Seitenkette eine Epoxygruppe enthält,
wobei das 1-Alken 7 bis 1 2 Kohlenstoffatome aufweist.

2. Verfahren nach Anspruch 1, bei dem der Mikroorganismus
(a) die Fähigkeit aufweist, das 1-Alken in eine Epoxyalkanverbindung zu epoxidieren und zu überführen;
(b) die Fähigkeit aufweist, die Epoxyalkanverbindung in eine epoxidierte Carbonsäure zu überführen; und
(c) die Fähigkeit aufweist, die epoxidierte Carbonsäure in den Polyester zu überführen.

3. Verfahren nach Anspruch 1, umfassend
ferner einen Schritt, bei dem der Mikroorganismus in einem Kulturmedium, das das 1-Alken enthält, gezüchtet wird.

4. Verfahren nach Anspruch 3, umfassend
ferner einen Schritt, bei dem der vom Mikroorganismus produzierte Polyester abgetrennt wird.

5. Verfahren nach Anspruch 4, bei dem
der Abtrennschritt das Gewinnen des Polyesters aus der Zelle des Mikroorganismus umfasst.

6. Verfahren nach Anspruch 1, bei dem
der Polyester in seinen Monomereinheiten zumindest 1 Mol-% einer Einheit der chemischen Formel (1) enthält: 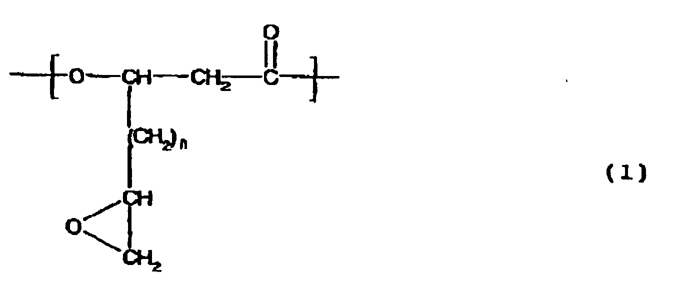 (worin n eine ganze Zahl von 1 bis 7 ist).

7. Verfahren nach Anspruch 6, bei dem
der Polyester in seinen Monomereinheiten zumindest 1 Mol-% einer Einheit der chemischen Formel (2) enthält: 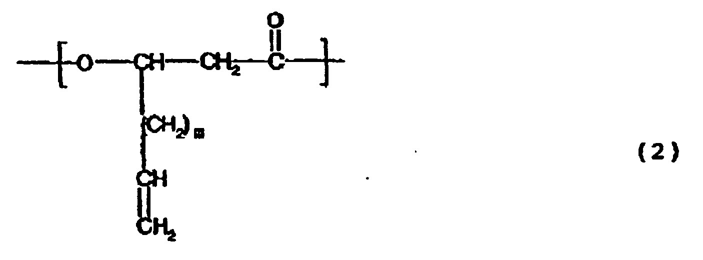 (worin m eine ganze Zahl von 1 bis 7 ist).

8. Verfahren nach Anspruch 6, bei dem
der Polyester in seinen Monomereinheiten zumindest 1 Mol-% einer Einheit der chemischen Formel (3) enthält: 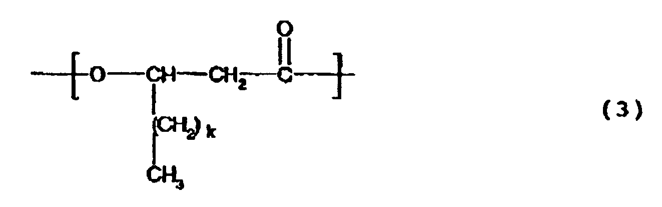 (worin k eine ganze Zahl von 0 bis 8 ist).

9. Verfahren nach Anspruch 1, bei dem
der Polyester ein Zahlenmittel des Molekulargewichts von 10.000 bis 1.000.000 aufweist.

10. Verfahren nach Anspruch 1, bei dem
der Mikroorganismus zur Gattung Pseudomonas gehört.

11. Verfahren nach Anspruch 10, bei dem
der Mikroorganismus Pseudomonas cichorii YN2, FERM BP-7375 ist.

12. Verfahren zur Herstellung eines vernetzten Polymers, umfassend einen Schritt, bei dem gemäß dem Verfahren nach Anspruch 1 ein Polyester erzeugt wird, und einen Schritt, bei dem der dadurch erhaltene Polyester durch die Reaktion des Polyesters mit einer Diaminverbindung vernetzt wird.

13. Verfahren nach Anspruch 12, bei dem
die Diaminverbindung Hexamethylendiamin ist.

14. Verfahren nach Anspruch 12, bei dem
die Reaktion bei einer Temperatur im Bereich von 50 bis 120°C erfolgt.

15. Verfahren nach Anspruch 12, bei dem
die Reaktion 10 bis 120 Minuten lang ausgeführt wird.

## Revendications

1. Procédé pour la production d'un polyester contenant un groupe époxy dans une de ses chaînes latérales, en utilisant un 1-alcène comme matière première, comprenant les étapes consistant
à mettre un 1-alcène en contact avec un micro-organisme présentant la capacité d'absorber le 1-alcène et de le convertir en un polyester, et
à convertir le 1-alcène en un polyester contenant un groupe époxy dans une de ses chaînes latérales, sous l'action du micro-organisme,
le 1-alcène ayant 7 à 12 atomes de carbone.

2. Procédé suivant la revendication 1, dans lequel le micro-organisme présente
(a) l'aptitude à époxyder et convertir le 1-alcène en un époxy-alcane ;
(b) l'aptitude à convertir l'époxy-alcane en un acide carboxylique époxydé ; et
(c) l'aptitude à convertir l'acide carboxylique époxydé en le polyester.

3. Procédé suivant la revendication 1, comprenant en outre l'étape de culture du micro-organisme dans un milieu de culture contenant le 1-alcène.

4. Procédé suivant la revendication 3, comprenant en outre l'étape d'isolement du polyester produit par le micro-organisme.

5. Procédé suivant la revendication 4, dans lequel l'étape d'isolement comprend la récupération du polyester à partir de la cellule du micro-organisme.

6. Procédé suivant la revendication 1, dans lequel le polyester contient au moins 1 % en moles d'un motif représenté par la formule chimique (1): 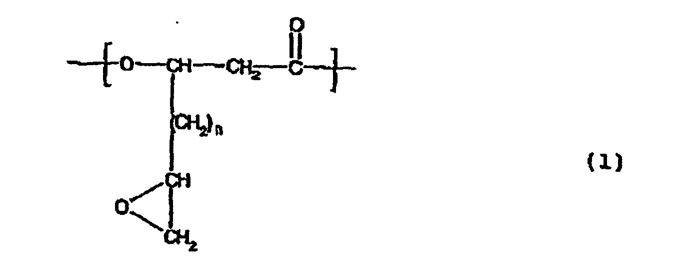 (dans laquelle n représente un nombre entier de 1 à 7) dans ses motifs monomères.

7. Procédé suivant la revendication 6, dans lequel le polyester contient au moins 1 % en moles d'un motif représenté par la formule chimique (2) : 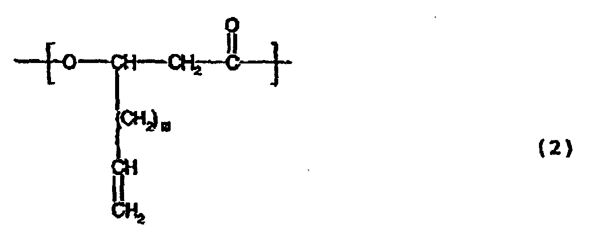 (dans laquelle m représente un nombre entier de 1 à 7) dans ses motifs monomères.

8. Procédé suivant la revendication 6, dans lequel le polyester contient au moins 1 % en moles d'un motif représenté par la formule chimique (3) : 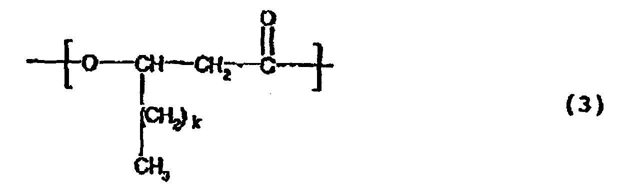 (dans laquelle k représente un nombre entier de 0 à 8) dans ses motifs monomères.

9. Procédé suivant la revendication 1, dans lequel le polyester a une moyenne en nombre du poids moléculaire de 10 000 à 1 000 000.

10. Procédé suivant la revendication 1, dans lequel le micro-organisme appartient à une espèce de Pseudomonas.

11. Procédé suivant la revendication 10, dans lequel le micro-organisme est le Pseudomonas cichorii YN2 ; FERM BP-7375.

12. Procédé pour la production d'un polymère réticulé, comprenant une étape de production d'un polyester suivant le procédé de la revendication 1 et une étape de réticulation du polyester ainsi obtenu en faisant réagir le polyester avec une diamine.

13. Procédé suivant la revendication 12, dans lequel la diamine est l'hexaméthylènediamine.

14. Procédé suivant la revendication 12, dans lequel la réaction est conduite à une température de 50°C à 120°C.

15. Procédé suivant la revendication 12, dans lequel la réaction est conduite pendant 10 minutes à 120 minutes.
